# EUROPEAN PATENT APPLICATION

(11) **EP 4 804 201 A1**
(43) Date of publication of application: **09.09.2026**
(21) Application number: 25161502.7
(22) Date of filing: 04.03.2025
(51) Int. Cl.: G16H 30/40

(54) **CONTEXT-SENSITIVE IMAGE QUALITY ASSESSMENT USING LANGUAGE-IMAGE MODELS**

(71) Applicant: Koninklijke Philips N.V., 5656 AE Eindhoven (NL)
(72) Inventor: SAALBACH, Axel, Eindhoven (NL); GROTH, Alexandra, Eindhoven (NL); MATUTE FLORES, Jose Alejandro, 5656AG Eindhoven (NL)
(74) Representative: Philips Intellectual Property & Standards

(57) **Abstract**

Disclosed is a computer-implemented method (100) for providing a value (208) of an image quality metric of at least one medical image (200) using a trained language-image model, the method comprising: receiving (102) the medical image (200) and medical context information (202); providing (104) the medical image (200) to a source encoder (204) configured to encode the medical image into an image embedding in an embedding space; generating (106) a text query (206) descriptive of the medical image (200); providing (108) the text query (206) to a query encoder (208) configured to encode the text query into a text embedding in the embedding space; determining (110) a similarity (210) between the image embedding and the text embedding; determining (112) the value (212) of the image quality metric using the similarity (210); and providing (114) the value (212) of the image quality metric.

## Description

### FIELD OF THE INVENTION

The invention relates to the field of medical imaging, and in particular to a quality assessment of medical images acquired by medical imaging.

### BACKGROUND OF THE INVENTION

In order to perform medical diagnoses based on medical images of a subject obtained using a medical imaging system, such as a magnetic resonance imaging system or a computed tomography system, the medical images need to undergo a quality assessment. Medical images showing deficiencies in image quality, for example noise, motion artifacts or insufficient coverage of an imaged area, may be unusable for diagnosing the subject or determining a subsequent treatment of the subject.

The document *"*AIGC Image Quality Assessment via Image-Prompt Correspondence" by Peng et al., published in the "Proceedings of the IEEE/CVF Conference on Computer Vision and Pattern Recognition Workshops. Vol. 6." held from the 17.06.2024 to the 21.06.2024 in Seattle, Washington, USA, describes an image quality assessment framework using artificial intelligence generated content (AIGC) images. Pre-trained image and text encoders of a contrastive language image pre-training (CLIP) model are used to encode the AIGC images and associated text prompts descriptive of the quality of the AIGC images into image and text encodings respectively. A correspondence between the image and text encodings in a shared embedding space is determined. The correspondence is calculated using a cosine similarity between the encodings. The resulting similarity is transformed into classification probabilities used for a subsequent regression task, wherein a quality score is obtained as an output of the regression task.

### SUMMARY OF THE INVENTION

In one aspect, the disclosure describes a computer-implemented method for providing a value of an image quality metric of at least one medical image using a trained language-image model. The method comprises receiving the medical image and medical context information associated with the medical image, wherein the medical context information is descriptive of a subject, a medical exam performed on the subject, and/or a medical imaging system which performed the medical exam to acquire the medical image. The method further comprises providing the medical image to a source encoder of the language-image model, wherein the source encoder is configured to encode the medical image into an image embedding in an embedding space. The method further comprises generating a text query descriptive of the medical image using the medical context information and/or the image quality metric. The method further comprises providing the text query to a query encoder of the language-image model, wherein the query encoder is configured to encode the text query into a text embedding in the embedding space. The method further comprises determining a similarity between the image embedding and the text embedding in the embedding space. The method further comprises determining the value of the image quality metric using the similarity. The method further comprises providing the value of the image quality metric.

In another aspect, the disclosure describes a computer system configured for providing a value of an image quality metric of at least one medical image using a trained language-image model, wherein the computer system comprises a memory unit storing machine executable instructions and the trained language-image model, wherein execution of the machine-readable instructions causes the computer system to receive the medical image and medical context information associated with the medical image, wherein the medical context information is descriptive of a subject, a medical exam performed on the subject, and/or a medical imaging system which performed the medical exam to acquire the medical image. Execution of the machine executable instructions further causes the computer system to provide the medical image to a source encoder of the language-image model, wherein the source encoder is configured to encode the medical image into an image embedding in an embedding space. Execution of the machine executable instructions further causes the computer system to generate a text query descriptive of the medical image using the medical context information and/or the image quality metric. Execution of the machine executable instructions further causes the computer system to provide the text query to a query encoder of the language-image model, wherein the query encoder is configured to encode the text query into a text embedding in the embedding space. Execution of the machine executable instructions further causes the computer system to determine a similarity between the image embedding and the text embedding in the embedding space. Execution of the machine executable instructions further causes the computer system to determine the value of the image quality metric using the similarity. Execution of the machine executable instructions further causes the computer system to provide the value of the image quality metric.

In another aspect, the disclosure describes a computer program product, wherein the computer program product comprises machine executable instructions configured for causing a computer system to execute the disclosed method.

### BRIEF DESCRIPTION OF THE DRAWINGS

In the following, examples are described in greater detail making reference to the drawings in which:
Fig. 1 is a flowchart for performing a computer-implemented method for providing a value of an image quality metric of at least one medical image using a trained language-image model.
Fig. 2A is a schematic of an exemplary use of the language-image model.
Fig. 2B is a schematic of an exemplary use of the language-image model using a context encoder.
Fig. 3A is a schematic of a medical imaging system communicatively coupled to a computer system.
Fig. 3B is a schematic of a medical imaging system communicatively coupled to a computer system, wherein the computer system is communicatively coupled to a further computer system.
Fig. 4 is a block diagram of an exemplary computer system for implementing at least part of the present method in accordance with an example of the present subject matter.

### DETAILED DESCRIPTION OF EMBODIMENTS

The present subject matter may advantageously enable a context-sensitive quality assessment of medical images, as a trained language-image model is used to assess the quality of the medical images using medical context information associated with the medical images. The trained language-image model may consider multiple attributes of image quality relevant to the quality assessment, wherein said multiple attributes of image quality may further differ based on a variable clinical context, for example based on different medical exams performed on a subject to obtained the medical images. The context-sensitive quality assessment may further differ for different medical imaging systems performing the medical exam, for example, medical images from a magnetic resonance imaging system may be assessed for different attributes of image quality than medical images from a computed tomography system. The present subject matter may therefore ensure a sufficient image quality of the medical images across a variety of clinical applications. In particular, the image quality of the medical images may be ensured to be sufficient for subsequent steps of a clinical workflow by providing images of sufficient quality for performing a diagnosis or determining a treatment plan for the subject.

The present subject matter may enable a quality assessment independent of individual assessments of various image quality metrics. For example, instead of providing the medical image to a set of separate algorithms dedicated to quantifying singular deficiencies in image quality such as an algorithm to detect insufficient coverage of an area to be imaged and a further algorithm to quantify a presence or absence of motion artifacts, the trained language-image model provides a similarity accounting for multiple attributes of image quality simultaneously. The present subject matter provides a value for the quality of the medical image in its medical context according to a context-sensitive image quality metric, wherein said value may avoid mixed quality assessments obtained from the set of separate algorithms, such as a positive assessment regarding the absence of motion artifacts and a negative assessment regarding the coverage.

Furthermore, the context-sensitive quality assessment may advantageously determine image quality in dependence of medical context information about the subject. For example, an absence of motion artifacts may be evaluated differently for an adult subject compared to a pediatric subject, as the pediatric subject may be more likely to move during the medical exam than the adult subject.

The present subject matter may further enable the context-sensitive quality assessment to be performed without requiring a database or list providing relationships between relevant attributes of image quality or image quality metrics quantifying said attributes to particular medical exams or medical imaging systems. Instead, the trained language-image model is configured to learn said relationships using the medical context information provided in addition to the medical images during a training of the trained language-image model.

The present subject matter may advantageously be applied to a variety of medical imaging systems, wherein the medical imaging system may be any one of a magnetic resonance imaging system, a computed tomography system, an x-ray system, a positron emission tomography system, a single photon emission tomography system, a digital fluoroscopy system, and a diagnostic ultrasound system. The present subject matter may further be advantageously applied to a variety of medical exams. Medical exams may exemplarily be performed for a detection of cancer, a detection of lesions, such as lesions in the brain, or a detection of a bone fracture.

According to one example, the generating of the text query comprises generating the text query based on a predefined template from a set of templates. The predefined template may be selected from the set of templates based on the medical context information. The predefined template preferably comprises a gap text, wherein gaps in the gap text are filled in by the medical context information.

The predefined templates may advantageously provide a consistent basis for determining the similarity of image embeddings of various medical images to text queries for a particular type of medical exam, thereby reducing a potential source of variation in the determined similarity. For example, a predefined template for a magnetic resonance imaging scan may indicate a level of image quality in relation to said type of medical exam, such as the text queries "A high quality image of a magnetic resonance imaging scan" or "A low quality image of a magnetic resonance imaging scan". The predefined template may further comprise references to a particular image quality metric, such as the text query "An image from a magnetic resonance imaging scan that is free of motion artifacts". Instead or alternatively, the predefined template may further comprise references to the medical context information, such as an age or gender of the subject or an area to be imaged in the medical exam, for example in the text query "A high quality image of a magnetic resonance imaging scan of the brain of a 70 year old male subject".

The predefined template may exemplarily be stored in a database, wherein the predefined templates may be loaded from the database during the execution of the method of the present subject matter. The predefined templates may comprise gap texts wherein specific medical context information may be filled in at predetermined gaps in the gap text. For example, a gap text "A high quality x-ray scan of [...] of a [...] year old [...] subject" in which the gaps are indicated as "[...]" may be filled in order of appearance of the gaps by medical context information concerning an area imaged by the medical exam, an age of the subject and a gender of the subject respectively.

The predefined templates may exemplarily be generated by an expert possessing relevant domain expertise, for example a physician or imaging specialist, in particular a radiologist, or a medical technical assistant. Alternatively, the predefined templates may be generated using a large language model. The predefined templates generated by the large language model may be reviewed and optionally modified by the expert, for example to more accurately reflect the available medical context information.

According to one example, the generating of the text query further comprises providing the text query to a user interface, and in response to the providing receiving modifications to the text query from the user interface, wherein the text query provided to the query encoder comprises the modifications.

Providing the text query to the user interface may advantageously enable an operator, for example an operator of the medical imaging system, to provide feedback by providing the modifications to the text query. The modifications may exemplarily be received during an ongoing medical exam, enabling a real-time adjustment of text queries.

According to one example, the language-image model further comprises a context encoder. According to this example, the method further comprises providing at least part of the medical context information to the context encoder, wherein the context encoder is configured to encode the medical context information into a context-information embedding in the embedding space. In this example, the encoding of the text embedding may further comprise combining the text embedding with the context-information embedding. In addition or alternatively, the encoding of the image embedding may further comprise combining the image embedding with the context-information embedding.

The use of the context encoder in addition to the source encoder and query encoder may enable a more accurate incorporation of the medical context information as the context encoder may be trained specifically on data having a data format common to the medical context information. For example, the source encoder may be trained on image data and the query encoder may be trained on text data, whereas the medical context information may comprise more complex data formats such as tabular data or time series data.

Furthermore, the context encoder enables an extending of the image embedding and/or the text embedding by additional information. For example, the image embedding may be extended by incorporating further medical context information which relates to the medical image but is not contained in the medical image itself, for example a pixel spacing or resolution of the medical image, or exam parameters of the medical exam used to acquire the medical image. Instead or alternatively, the text embedding may be extended further by incorporating medical context information which relates to the text query but could not be extracted from the medical context information using the query encoder. For example, medical context information may be provided to the query encoder as a medical report, in particular a radiology report, wherein the radiology report comprises tabular data.

In a further example, the combining exemplarily comprises determining a weighted sum of the text embedding and/or the image embedding with the context-information embedding, and wherein the text embedding and/or the image embedding used for determining the similarity comprises the weighted sum.

This combining using a weighted sum may enable the extending of the image embedding and/or the text embedding to be performed while keeping the architectures of the source encoder, query encoder and context encoder separate. Keeping the architectures of the encoders separate may be advantageous as each encoder may be trained on specific data types only, such as the source encoder being trained only on image data.

According to one example, the generating of the text query comprises generating a set of text queries, wherein text queries in the set of text queries are descriptive of different image quality metrics, different levels of image quality and/or comprise different amounts of the medical context information. In this example, the set of text queries is provided to the query encoder, resulting in a set of text embeddings, wherein the determining of the similarity comprises determining a set of similarities between the medical image and the set of text embeddings. The providing of the value of the image quality metric may exemplarily comprise providing an extremal value of the image quality metric determined using an extremal similarity in the set of similarities. In another example, the providing of the value of the image quality metric comprises providing an aggregated value of the image quality determined using an aggregation of the similarities in the set of similarities. The aggregation may exemplarily comprise determining an average or median of the similarities in the set of similarities.

A set of text queries may exemplarily be descriptive of different image quality metrics. Said set of text queries may comprise the text queries "An image of an x-ray scan free of motion artifacts", "An image of an x-ray scan free of noise", "An image of an x-ray scan of sufficient contrast". Said set of text queries may result in a set of similarities indicating a high similarity of the medical image to the text queries "An image of an x-ray scan free of motion artifacts" and "An image of an x-ray scan free of noise" and a low similarity to the text query "An image of an x-ray scan of sufficient contrast". The aggregation may exemplarily comprise providing the minimum of the set of similarities as the value of the image quality metric. This may advantageously result in the medical image being used for subsequent clinical workflows only in case the medical image is determined to be of sufficient quality according to all queried image quality metrics.

A set of text queries may in a further example be descriptive of different levels of image quality. Said set of text queries may comprise the text queries "An image of an x-ray scan showing no deficiencies in image quality", "An image of an x-ray scan showing minor deficiencies in image quality" and "An image of an x-ray scan showing major deficiencies in image quality". In one example, the provided value of the image quality metric may be determined using an extremal similarity in the set of similarities, for example the highest similarity in the set of similarities. For example, in case the highest similarity is associated with the text query "An image of an x-ray scan showing no deficiencies in in image quality", the value of the image quality metric may be determined to be higher than in case of the highest similarity being associated with the text query "An image of an x-ray scan showing major deficiencies in image quality". In another example, the providing of the image quality metric may comprise applying a softmax function to the similarities in the set of similarities, resulting in a set of probabilities. The provided value of the image quality metric may then exemplarily be determined using the set of probabilities, in particular using an extremal value of the set of probabilities. For example, if the highest probability in the set of probabilities is associated with the text query "An image of an x-ray scan showing no deficiencies in in image quality", the value of the image quality metric may be determined to be higher than in case of the highest probability being associated with the text query "An image of an x-ray scan showing major deficiencies in image quality". This may advantageously provide a more accurate assessment of the image quality of the medical image compared to a similarity obtained using a single text query descriptive of a single level of image quality.

A set of text queries may in a further example be descriptive of different amounts of the medical context information. Said set of text queries may comprise the text queries "An image free of motion artifacts", "An image of an x-ray scan free of motion artifacts", "An image of an x-ray scan of an arm, wherein the image is free of motion artifacts", "An image of an x-ray scan of an arm of a 10 year old subject, wherein the image is free of motion artifacts". In this example, the similarity corresponding to the last text query may be higher than the similarities corresponding to the other text queries in the set of text queries. This may advantageously indicate that while the medical image is not free of motion artifacts, said motion artifacts are present to a degree that is expected when imaging a pediatric subject.

According to one example, the method further comprises comparing the provided value of the image quality metric to an image quality threshold, wherein the image quality threshold is determined at least in part based on the medical context information.

The image quality threshold advantageously may serve as a criterion for deciding whether the medical image is of sufficient quality for use in subsequent clinical workflows. The image quality threshold may be set in dependence of the medical context information. For example, in case of the medical context information indicating a medical exam performed to detect cancer, the image quality threshold may be set to a value corresponding to a higher image quality than in a case of the medical context information indicating a medical exam performed to detect a bone fracture in an arm.

According to one example, the method further comprises generating a quality evaluation in response to the value of the image quality metric not satisfying a comparison criterion set by the image quality threshold, wherein the quality evaluation is descriptive of deficiencies in image quality of the medical image according to the comparison of the value of the image quality metric with the image quality threshold. In this example, the method further comprises providing the quality evaluation.

The quality evaluation may advantageously aid in interpreting the provided value of the image quality metric. For example, the quality evaluation may comprise an explanatory statement stating the following: "The image is free of motion artifacts and the coverage is sufficient. However, the contrast of the image is low". Such an explanatory statement may aid in determining how the quality of subsequently acquired medical images may be improved, for example by repeating the medical exam in such a way as to increase the contrast.

The quality evaluation may comprise a text, a Boolean value indicating sufficient or insufficient image quality, a number, in particular the value of the image quality metric, a visual notification, for example a green or red light indicating sufficient or insufficient image quality respectively, and/or an audio signal, in particular a speech signal. The visual notification may exemplarily be provided on a display of the medical imaging system or on a display of a computer system communicatively coupled to the medical imaging system.

According to one example, the medical image and the medical context information are received from the medical imaging system, and wherein the method further comprises controlling the medical imaging system based on the comparing.

In this example, a real-time application of the method of the present subject matter is enabled while the subject is still present at a site of the medical imaging system. The controlling advantageously enables a feedback to be provided to the medical imaging system used to acquire said medical images based on the value of the image quality metric. Thus, deficiencies in image quality may be corrected without requiring a recall of the subject to repeat the medical exam at a later time.

According to one example, the controlling comprises modifying exam parameters of the medical exam in response to the value of the image quality metric not satisfying the comparison criterion set by the image quality threshold. The exam parameters exemplarily comprise any one of an exam duration, an x-ray tube voltage, an x-ray tube current, an exposure time, a slice thickness, a magnetic field strength, a pulse sequence, and/or a field of view parameter. The exam parameters may differ depending on the medical imaging system, for example, the exam parameters available for a magnetic resonance imaging system may be different from the exam parameters available for a computed tomography system. The exam parameters may further differ for particular medical exams performed using the medical imaging system. Instead or in addition, the controlling may further comprise instructing the medical imaging system to repeat the medical exam in response to the value of the image quality metric not satisfying the comparison criterion set by the image quality threshold.

The modifying may thereby advantageously enable an automatic correction of the exam parameters, which may reduce a time spent before the medical exam can be repeated compared to a manual correction of the exam parameters by an expert operating the medical imaging system. Furthermore, the modifying may result in exam parameters more suitable to obtaining a medical image of sufficient quality compared to exam parameters set by the expert operating the medical imaging system.

According to one example, the modifying comprises a modifying based on the medical exam and/or a difference between the value of the image quality metric and the image quality threshold. A modification in a value of an exam parameter may preferably be proportional to the difference between the value of the image quality metric and the image quality threshold. For example, a larger difference between the value of the image quality metric and the image quality threshold may result in a larger modification of a particular exam parameter than a smaller difference between the value of the image quality metric and the image quality threshold. The modifying may thereby reduce a number of repetitions needed before a medical image of sufficient quality is obtained.

The instructing of the medical imaging system to repeat the medical exam may exemplarily comprise providing suggested exam parameters, wherein the suggested exam parameters are preferably provided to a display of the medical imaging system or a display of a computer system communicatively coupled to the medical imaging system. In response to the providing of the suggested exam parameters, a confirmation may be received and the medical exam may be repeated using the suggested exam parameters following the receiving of the confirmation. Alternatively, the confirmation may further comprise modifications to the suggested exam parameters may be received, wherein the medical exam may be repeated using the modifications to the suggested exam parameters. The confirmation and optionally the modifications to the suggested exam parameters may preferably be provided by an operator of the medical imaging system. In another example, the medical exam may be repeated directly using the suggested exam parameters without requiring the confirmation.

According to one example, the controlling comprises enabling a providing of the medical image to a diagnosis tool in response to the value of the image quality metric satisfying the comparison criterion set by the image quality threshold, and in response receiving from the diagnosis tool a medical diagnosis based on the medical image. In this example, the method optionally further comprises providing the medical context information to the medical diagnosis tool for performing the medical diagnosis.

The controlling according to this example may advantageously enable or disable a providing of the medical image to the diagnosis tool depending on a minimum image quality required by the diagnosis tool, wherein said minimum image quality corresponds to the comparison criterion set by the image quality threshold. Thereby, a probability of receiving an erroneous diagnosis from the diagnosis tool may be reduced as medical images of insufficient quality are prohibited from being provided as input to the diagnosis tool.

According to one example, the method further comprises a further training of the language-image model using the medical image and the text query.

The further training may advantageously enable the trained language-image model to be fine-tuned to the needs or preferences of a particular entity using the trained language-image model, such as a particular hospital, clinic or a department in said hospital or clinic. In particular, the fine-tuning may comprise fine-tuning the trained language-image model to a particular type of medical images and/or a particular medical context. For example, the trained language-image model may be used differently in a cardiology department compared to a neurology department of a clinic. The cardiology department may preferentially use a medical imaging system for acquiring medical images of the heart while the neurology department preferentially uses a medical imaging system for acquiring medical images of the brain. Different image quality metrics may be used to assess the quality of the medical images of the heart than are used to assess the quality of the medical images of the brain. Furthermore, the medical context information may differ between the cardiology department and the neurology department. For example, the cardiology department may use different medical exams and/or exam parameters than the neurology department. The trained language-image model may then be provided with additional paired data of medical images and text queries generated based on the medical context information, advantageously increasing the ability of the trained language-image model to identify relevant features in medical images of the heart or medical images of the brain respectively.

### Training of the language-image model:

The trained language-image model may exemplarily be provided as a fully trained model. Alternatively, the trained language-image model may be obtained by training an untrained language-image model. Training of the untrained language-image model comprises training the source encoder, the query encoder and optionally the context encoder. The term "untrained language-image model" as used herein further encompasses partially trained or pre-trained models on which further training is performed.

In one example, the untrained language-image model comprises a contrastive language-image pre-training, CLIP, model. The CLIP model may be configured to encode images into image embeddings using the source encoder and to encode texts into text embeddings using the query encoder, wherein the image embeddings and the text embeddings are defined in a shared embedding space. The encoding may be configured such that image embeddings of higher quality images are closer to text embeddings descriptive of said higher quality images than to text embeddings of lower quality images. Similarly, the encoding may be configured such that image embeddings of lower quality images are closer to text embeddings descriptive of said lower quality images than to text embeddings of higher quality images.

The dimension of the embedding space may exemplarily be determined by the architecture of the source encoder and/or query encoder. The dimension of the embedding space may further be determined features of the medical images, for example the size or resolution of the medical images. The dimension of the embedding space may further be determined by features of the text queries, for example a length of the text queries.

In one example, the source encoder comprises a neural network, preferably a convolutional neural network, CNN, or a vision transformer, ViT. The neural network of the source encoder may further comprise a text encoder. In another example, the query encoder comprises a neural network, preferably a transformer model. In yet another example, the context encoder comprises a neural network, wherein the architecture of said neural network of the context encoder is chosen based on the medical context information in the medical reports. For example, the neural network of the context encoder may comprise a TabNet architecture in case the medical reports comprise tabular data. In another example, the neural network of the context encoder may comprise a long short-term memory network architecture or a temporal convolutional network architecture.

Training of the untrained language-image model exemplarily comprises providing paired data to the untrained language-image model, wherein the paired data comprises a medical image and an associated text query. The text query may for example be extracted from a medical report, in particular a radiology report, comprising medical context information. The extracting of the text query from the medical report exemplarily comprises recognizing text in the medical report by applying an optical character recognition algorithm and extracted the recognized text. The medical context information extracted from the medical report may in particular comprise information relating to a quality of a medical image associated with the medical report.

The training may be performed by providing two sets of images to the untrained language-image model, wherein the two sets of images share a particular medical context, for example, images in both of the two sets may be magnetic resonance images of the brain acquired using identical exam parameters or exam parameters differing by less than a predefined maximal difference. The images in the two sets of images differ in that images in a first of the two sets comprise a particular deficiency in image quality, for example insufficient coverage of the brain, while images in a second of the two sets of images are free of said deficiency. The images in the first set may then be paired with a text query descriptive of a general or specific deficiency in image quality, such as the text queries "Bad magnetic resonance image" or "Magnetic resonance image with insufficient coverage". The images in the second set may then be paired with a text query descriptive of a general or specific lack of said deficiency in image quality, such as the text queries "Good magnetic resonance image" or "Magnetic resonance image with sufficient coverage". Said text queries may be generated using the medical context information extracted from the medical report or may alternatively be provided by manual annotation of the medical images by an expert possessing the relevant domain expertise.

The training may comprise providing the medical images in the first and second set of images to the source encoder while providing the text queries generated using the medical context information associated with said images in the first and second set of images to the query encoder. Furthermore, medical context information relating to the medical images, for example a pixel size or a field strength used for acquiring the medical image, may be provided to the source encoder. The training may further comprise providing the medical reports to the context encoder. Training the neural networks of the source encoder, the query encoder and/or the context encoder may comprise evaluating a loss function and adjusting weights and/or biases of said neural networks using standard techniques such as gradient-based optimization. The loss function may preferably comprise a contrastive loss function, for example a noise contrastive estimation function, InfoNCE. In another example, the loss function may comprise a triplet loss, a mean squared error or a mean absolute error. The performance of any one of the neural networks can be optimized using techniques like early stopping, learning rate scheduling, dropout, and regularization to prevent overfitting.

The medical images used for the training may be stored in a medical database of medical images and loaded from said medical database to perform the training. The medical reports comprising the medical context information used for generating the text queries paired to said medical images may be stored in the same medical database as the medical images or a further medical database and loaded from said medical database or further medical database to perform the training.

The training may exemplarily be performed using a training data set comprising medical reports and associated medical images. A particular medical report in the training data set may be associated with multiple medical images, for example if the multiple medical images have been acquired during a single medical exam. The training data set may be generated by loading the medical images and/or the medical reports from the medical database and optionally by loading the medical reports from the further medical database. The source encoder, query encoder and/or context encoder may comprise pre-trained neural networks, reducing the required size of the training dataset.

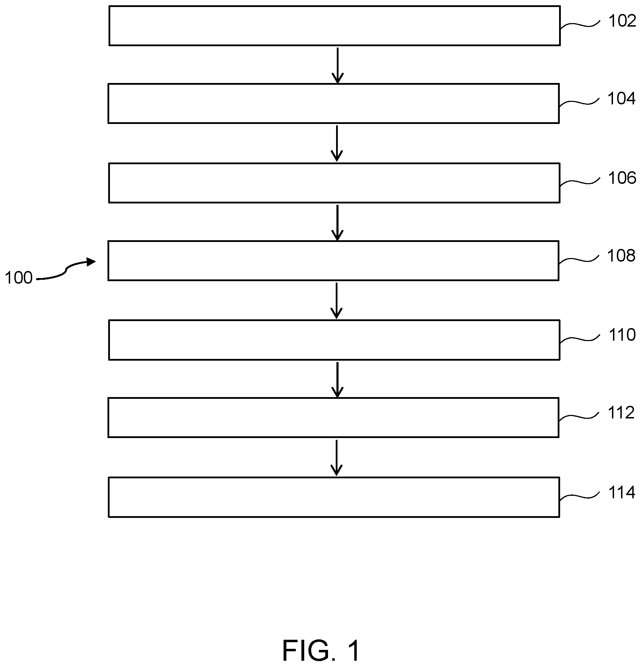

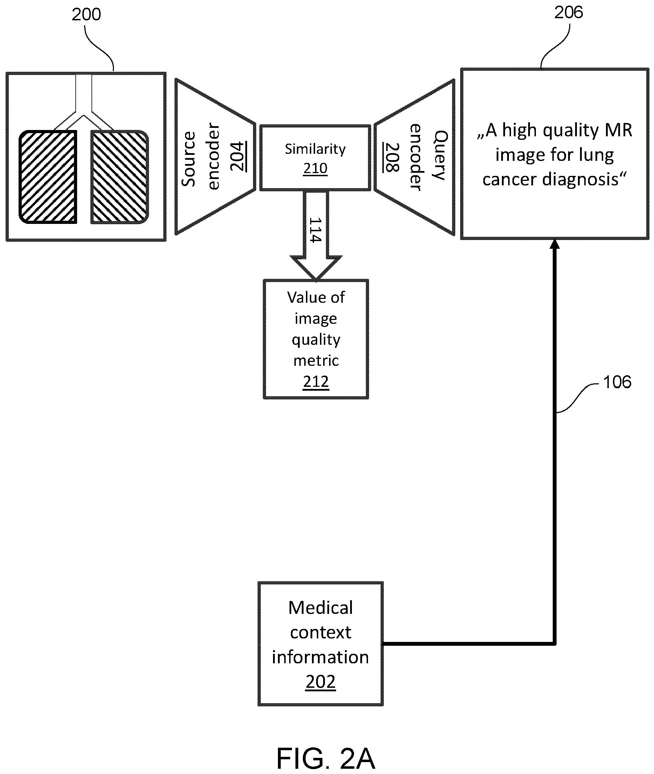

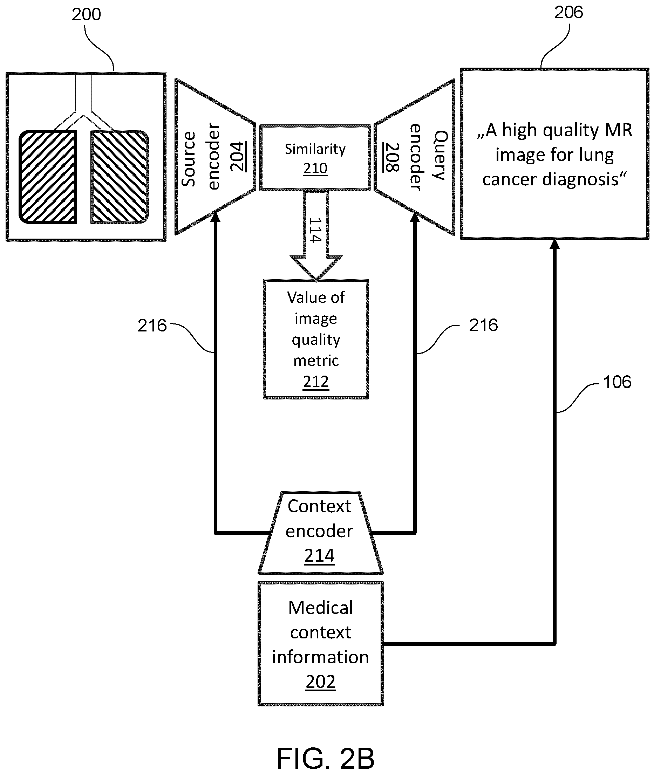

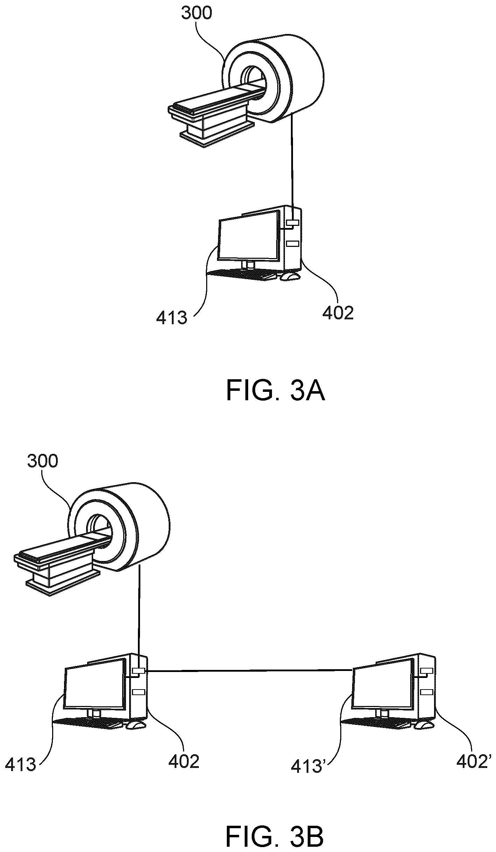

Fig. 1 shows a flowchart for performing a computer-implemented method 100 for providing a value 212 of an image quality metric of at least one medical image 200 using a trained language-image model. Examples described in relation to Fig. 1 may be combined with examples described in relation to any one of the following figures Fig. 2A, Fig. 2B, Fig. 3A or Fig. 3B.

For example, the image quality metric may comprise a metric quantifying attributes of image quality such as a presence or absence of noise, a presence or absence of motion artifacts, a coverage of an area imaged by the medical exam, an image contrast, an image sharpness, an image contrast or an image resolution. Furthermore, the image quality metric may account for multiple attributes of quality in a context-sensitive manner, wherein the relevant attributes of quality are learned by the trained language-image model during the training.

In step 102 of the method 100, a medical image and medical context information associated with the medical image is received, wherein the medical context information is descriptive of a subject, a medical exam performed on the subject, and/or a medical imaging system which performed the medical exam to acquire the medical image.

The medical image may comprise a 2D image or a 3D image. The medical image may be received directly from the medical imaging system, in particular while the medical exam is ongoing. Alternatively, the medical image may be stored in a memory unit or database of a computer system and received from said memory unit or database, in particular after the medical exam has been completed.

The medical context information may exemplarily comprise information about an age of the subject, a gender of the subject, a medical history of the subject, and/or laboratory values of the subject, for example laboratory values acquired by evaluating medical tests performed on the subject before the performing of the medical exam. The medical context information may further comprise information about the medical exam, such as an imaging protocol, in particular a pulse sequence used in a medical resonance imaging protocol or a modality used in a computed tomography scan, the modality for example indicating a helical scan. The medical context information may further comprise exam parameters used by the medical imaging system during the performing of the medical exam. Said exam parameters may exemplarily comprise x-ray tube current or x-ray tube voltage parameters or other parameters relating to a measure of radiation received by the subject in a computed tomography scan, a magnetic field strength used in a magnetic resonance imaging scan, a contrastive agent and optionally a dose of said contrastive agent used in a magnetic resonance imaging scan, and/or a field of view parameter. The medical context information may further comprise text information specifying a label or title of the medical exam, for example "Helical CT scan of torso".

In step 104 of the method 100, the medical image is provided to a source encoder of the language-image model, wherein the source encoder is configured to encode the medical image into an image embedding in an embedding space. The source encoder may exemplarily comprise a convolutional neural network or a vision transformer. The source encoder may further comprise a text transformer. In one example, the providing 104 comprises providing an image space representation of the medical image to the source encoder. In another example, the providing 104 comprises providing a frequency space representation of the medical image to the source encoder. Said frequency space representation may be acquired as a result of a Fourier transformation applied to the image space representation of the medical image.

In step 106 of the method 100, a text query descriptive of the medical image is generated using the medical context information and/or the image quality metric. In one example, the text query describes the medical image using the image quality metric, for example as "An image free of motion artifacts" or "An image affected by motion artifacts". In another example, the text query describes the medical image using the medical context information, for example as "A high quality CT image" or "A low quality CT image". In yet another example, the text query describes the medical image using both the medical context information and the image quality metric, for example as "A CT image free of motion artifacts" or "A CT image affected by motion artifacts".

In step 108 of the method 100, the text query is provided to a query encoder of the language-image model, wherein the query encoder is configured to encode the text query into a text embedding in the embedding space. The query encoder may exemplarily comprise a transformer neural network model. In another example, the query encoder may use word embedding techniques such as TF-IDF embedding or Word2Vec embedding.

In step 110 of the method 100, a similarity is determined between the image embedding and the text embedding in the embedding space. The image embedding and the text embedding may be represented as vectors in the embedding space, wherein a dimension of the vector representing the image embedding is identical to a dimension of the vector representing the text embedding. The determining 110 may exemplarily determining the similarity as a result of a dot product, cosine similarity, Euclidean distance, Manhattan distance or Mahalanobis distance between the vectors representing the image embedding and the text embedding. The determining 110 may further comprise additional normalization or rescaling operations applied to the similarity.

In step 112 of the method 100, the value of the image quality metric is determined using the similarity. In one example, the value of the image quality metric is identical to the similarity. In another example, the image quality metric is determined by rescaling the similarity to a predetermined interval, for example the interval [0,1], wherein a higher value of the image quality metric in said interval may exemplarily be indicative of higher image quality of the medical image. In another example, the value of the image quality metric may be determined using a set of similarities corresponding to a set of medical images, for example a set of medical images acquired during a particular medical exam. Alternatively, the set of similarities may correspond to different text queries associated with a single medical image. The value of the image quality metric may exemplarily be determined as an extremal value, an average or a median of similarities in the set of similarities.

In step 114 of the method 100, the value of the image quality metric is provided. The providing 114 may comprise a providing of the value to a user interface, in particular to a user interface of the medical imaging system or to a display of a computer system communicatively coupled to the medical imaging system. Instead or additionally, the providing may further comprise providing the value of the image quality metric to tools used in a subsequent clinical workflow, such as diagnosis tools. The providing 114 may comprise providing a single numeric value, a vector or a matrix. For example, in case of the image quality metric quantifying the presence or absence of motion artifacts, a three-dimensional vector may be provided specifying the presence or absence of the motion artifacts along different spatial dimensions.

Fig. 2A shows a schematic of an exemplary use of the language-image model. Any examples described in relation to Fig. 2A likewise apply to examples described in relation to Fig. 2B.

The medical image 200 as shown in Fig. 2A exemplarily shows a medical image of the lungs of a subject. The medical context information 202 may comprise textual information extracted from a medical report, in particular a radiology report, wherein the radiology report is associated with a medical exam that was performed to acquire the medical image 200. Said radiology report may indicate a modality of the medical exam, in this example a magnetic resonance imaging exam, and a purpose of the medical exam, in this example to detect a presence of cancer in the lungs of the subject. The text query 206 may then be generated 106 to incorporate said modality and said purpose, resulting in the text query "A high quality MR image for lung cancer diagnosis".

The medical image 200 of the lungs of the subject is provided to the source encoder 204, resulting in an image embedding of the medical image 200. The text query 206 is provided to the query encoder 208, resulting in a text embedding of the text query 206. A similarity 210 is determined using the image embedding and the text embedding. The similarity 210 is then used to determine the value 212 of the image quality metric and provide 114 the value of the image quality metric.

Fig. 2B shows a schematic of an exemplary use of the language-image model using a context encoder 214.

In Fig. 2B, in addition to the use of the medical context information 202 for generating 106 the text query 206 as described in relation to Fig. 2A, the medical context information 202 may further be provided to the context encoder 214. The context encoder 214 may exemplarily encode medical context information 202 relating to the medical image 200, for example a resolution of the medical image 200, in a context embedding of the image, and combine 216 the context embedding of the image with the image embedding provided by the source encoder 204. Instead or alternatively, the context encoder 214 may exemplarily encode medical context information 202 relating to the text query 206, for example an age of the subject being 70 and a gender of the subject being male, in a context embedding of the text, and combine 216 the context embedding of the text with the text embedding.

Fig. 3A shows a schematic of a medical imaging system 300 communicatively coupled to a computer system 402. Any examples described in relation to Fig. 3A may in particular be combined with examples described in relation to Fig. 3B. The computer system 402 may exemplarily be integrated into the medical imaging system 300. The computer system 402 may comprise a display 413. The display 413 may be integrated into the medical imaging system 300. Alternatively, the display 413 may comprise a screen connected to the computer system 402. The display 413 may be configured to display a system console of the medical imaging system 300. The system console may be configured to display medical images acquired by the medical imaging system 300. The system console may further be configured to provide an interface for controlling the medical imaging system 300. The trained language-image model may be stored on the computer system 402. The computer system 402 may be configured to perform the disclosed method. The computer system 402 may comprise the medical database storing the medical images and optionally the further medical database storing the medical context information.

Fig. 3B shows a schematic of a medical imaging system 300 communicatively coupled to a computer system 402, wherein the computer system 402 is communicatively coupled to a further computer system 402'. In one example, the further computer system 402' is a computer system possessing larger computational resources than the computer system 402, for example increased processing capacities or increased storage capacities. The further computer system 402' may for example comprise a high-performance computer system or a server accessed via a cloud service. In one example, the trained language-image model may be stored on the further computer system 402'. According to this example, the computer system 402 provides the medical image and the medical context information to the further computer system 402, wherein the further computer system 402' is configured to perform the disclosed method. According to this example, the computer system 402 is further configured to receive the value of the image quality metric from the further computer system 402'.

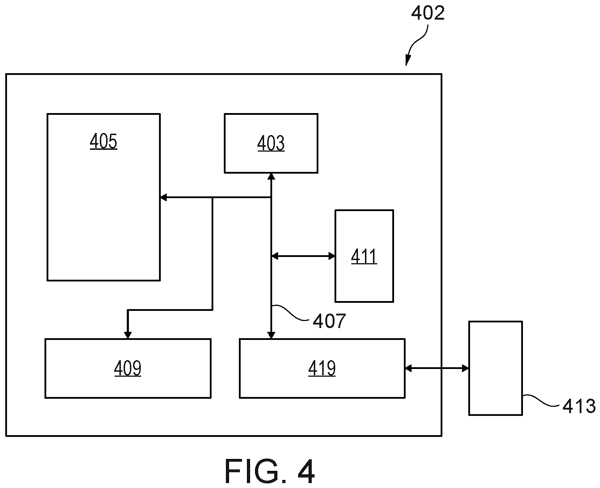

Fig. 4 is a block diagram of an exemplary computer system 402 for implementing the present method in accordance with an example of the present subject matter. The components of the computer system 402 may include, but are not limited to, one or more processors or processing units 403, a storage system 411, a memory unit 405, and a bus 407 that couples various system components including memory unit 405 to processor 403. The storage system 411 may include for example a hard disk drive (HDD). The memory unit 405 may include computer system readable media in the form of volatile memory, such as random access memory (RAM) and/or cache memory.

The computer system 402 may also communicate with one or more external devices such as a keyboard, a pointing device, a display 413, etc.; one or more devices that enable a user to interact with computer system 402; and/or any devices (e.g., network card, modem, etc.) that enable the computer system 402 to communicate with one or more other computing devices. Such communication can occur via I/O interface(s) 419. Still yet, the computer system 402 can communicate with one or more networks such as a local area network (LAN), a general wide area network (WAN), and/or a public network (e.g., the Internet) via a network adapter 409. As depicted, the network adapter 409 communicates with the other components of the client system 402 via bus 407.

The memory unit 405 is configured to store applications that are executable on the processor 403. For example, the memory unit 405 may comprise an operating system as well as one or more application programs. The application programs comprise instructions that when executed enable to perform the method described before, e.g. with reference to Fig. 1.

As will be appreciated by one skilled in the art, aspects of the present disclosure may be implemented as an apparatus, method, computer program or computer program product. Accordingly, aspects of the present disclosure may take the form of an entirely hardware implementation, an entirely software implementation (including firmware, resident software, micro-code, etc.) or an implementation combining software and hardware aspects that may all generally be referred to herein as a "circuit," "module" or "system." Furthermore, aspects of the present disclosure may take the form of a computer program product embodied in one or more computer readable medium(s) having computer executable code embodied thereon. A computer program comprises the computer executable code or "program instructions".

The term "computer system" refers to data processing hardware and encompasses all kinds of apparatus, devices, and machines for processing data, including by way of example, a programmable processor, a computer, or multiple processors or computers. The apparatus can also be or further include special purpose logic circuitry, e.g., a central processing unit (CPU), a FPGA (field programmable gate array), or an ASIC (application specific integrated circuit). In some implementations, the data processing apparatus and/or special purpose logic circuitry may be hardware-based and/or software-based. The apparatus can optionally include code that creates an execution environment for computer programs, e.g., code that constitutes processor firmware, a protocol stack, a database management system, an operating system, or a combination of one or more of them. The present disclosure contemplates the use of data processing apparatuses with or without conventional operating systems, for example LINUX, UNIX, WINDOWS, MAC OS, ANDROID, IOS or any other suitable conventional operating system.

Any combination of one or more computer readable medium(s) may be utilized. The computer readable medium may be a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device.

'Computer memory' or 'memory' is an example of a computer-readable storage medium. Computer memory is any memory which is directly accessible to a processor. 'Computer storage' or 'storage' is a further example of a computer-readable storage medium. Computer storage is any nonvolatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present disclosure. Computer executable code for carrying out operations for aspects of the present disclosure may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

Generally, the program instructions can be executed on one processor or on several processors. In the case of multiple processors, they can be distributed over several different entities. Each processor could execute a portion of the instructions intended for that entity. Thus, when referring to a system or process involving multiple entities, the computer program or program instructions are understood to be adapted to be executed by a processor associated or related to the respective entity.

It is understood that one or more of the aforementioned examples may be combined as long as the combined examples are not mutually exclusive.

While the invention has been illustrated and described in detail in the drawings and foregoing description, such illustration and description are to be considered illustrative or exemplary and not restrictive; the invention is not limited to the disclosed examples.

### REFERENCE SIGNS LIST

- 100: method
- 102, 104, 106, 108, 110, 112, 114: method steps
- 200: medical image
- 202: medical context information
- 204: source encoder
- 206: text query
- 208: query encoder
- 210: similarity
- 212: value of image quality metric
- 214: context encoder
- 216: combining embeddings
- 300: medical imaging system
- 402, 402': computer system
- 403: processors
- 405: memory unit
- 407: bus
- 409: network adapter
- 411: storage system
- 413, 413': external devices
- 419: I/O-Interface

## Claims

1. A computer-implemented method (100) for providing a value (208) of an image quality metric of at least one medical image (200) using a trained language-image model, the method comprising:
- receiving (102) the medical image (200) and medical context information (202) associated with the medical image, wherein the medical context information is descriptive of a subject, a medical exam performed on the subject, and/or a medical imaging system (300) which performed the medical exam to acquire the medical image;
- providing (104) the medical image (200) to a source encoder (204) of the language-image model, wherein the source encoder is configured to encode the medical image into an image embedding in an embedding space;
- generating (106) a text query (206) descriptive of the medical image (200) using the medical context information (202) and/or the image quality metric;
- providing (108) the text query (206) to a query encoder (208) of the language-image model, wherein the query encoder is configured to encode the text query into a text embedding in the embedding space;
- determining (110) a similarity (210) between the image embedding and the text embedding in the embedding space;
- determining (112) the value (212) of the image quality metric using the similarity (210); and
- providing (114) the value (212) of the image quality metric.

2. The method (100) according to claim 1, wherein the generating (106) of the text query (206) comprises generating the text query based on a predefined template from a set of templates, wherein the predefined template is selected from the set of templates based on the medical context information (202), wherein the predefined template preferably comprises a gap text, wherein gaps in the gap text are filled in by the medical context information (202).

3. The method (100) according to any one of claims 1 or 2, wherein the generating (106) of the text query (206) further comprises providing the text query to a user interface, and in response to the providing receiving modifications to the text query from the user interface, wherein the text query provided to the query encoder (208) comprises the modifications.

4. The method (100) according to any one of the preceding claims, wherein the language-image model further comprises a context encoder (214), wherein the method further comprises:
- providing at least part of the medical context information (202) to the context encoder (214), wherein the context encoder is configured to encode the medical context information (202) into a context-information embedding in the embedding space,
and wherein any one of the following applies:
- the encoding of the text embedding further comprises combining (216) the text embedding with the context-information embedding,
- the encoding of the image embedding further comprises combining (216) the image embedding with the context-information embedding,
and wherein the combining exemplarily comprises determining a weighted sum of the text embedding and/or the image embedding with the context-information embedding, and wherein the text embedding and/or the image embedding used for determining (112) the similarity (210) comprises the weighted sum.

5. The method (100) according to any one of the preceding claims, wherein the generating (106) of the text query (206) comprises generating a set of text queries, wherein text queries in the set of text queries are descriptive of different image quality metrics, different levels of image quality and/or comprise different amounts of the medical context information (202), wherein the set of text queries is provided to the query encoder, resulting in a set of text embeddings,
wherein the determining (112) of the similarity (210) comprises determining a set of similarities between the medical image and the set of text embeddings, and
wherein the providing (114) of the value (212) of the image quality metric comprises any one the following:
- providing an extremal value of the image quality metric determined using an extremal similarity in the set of similarities;
- providing an aggregated value of the image quality metric determined using an aggregation of the similarities in the set of similarities, the aggregation exemplarily comprising an average or median.

6. The method (100) according to any one of the preceding claims, wherein the method further comprises comparing the provided value (212) of the image quality metric to an image quality threshold, wherein the image quality threshold is determined at least in part based on the medical context information (202).

7. The method (100) according to claim 6, wherein the method further comprises:
- generating a quality evaluation in response to the value (212) of the image quality metric not satisfying a comparison criterion set by the image quality threshold, wherein the quality evaluation is descriptive of deficiencies in quality of the medical image (200) according to the comparison of the value (212) of the image quality metric with the image quality threshold; and
- providing the quality evaluation.

8. The method (100) according to any one of claims 6 or 7, wherein the medical image (200) and the medical context information (202) are received from the medical imaging system (300), and wherein the method further comprises controlling the medical imaging system (300) based on the comparing.

9. The method (100) according to claim 8, wherein the controlling comprises modifying exam parameters of the medical exam in response to the value (212) of the image quality metric not satisfying the comparison criterion set by the image quality threshold, wherein the exam parameters exemplarily comprise any one of an exam duration, an x-ray tube voltage, an x-ray tube current, an exposure time, a slice thickness, a magnetic field strength, a pulse sequence, and/or a field of view parameter.

10. The method (100) according to any one of claims 8 or 9, wherein the modifying comprises a modifying based on the medical exam and/or a difference between the value (212) of the image quality metric and the image quality threshold.

11. The method (100) according to any one of claims 8 to 10, wherein the controlling comprises instructing the medical imaging system (300) to repeat the medical exam in response to the value (212) of the image quality metric not satisfying the comparison criterion set by the image quality threshold.

12. The method (100) according to any one of claims 8 to 11, wherein the controlling comprises enabling a providing of the medical image (200) to a diagnosis tool in response to the value (212) of the image quality metric satisfying the comparison criterion set by the image quality threshold, and in response receiving from the diagnosis tool a medical diagnosis based on the medical image (200), wherein the method optionally further comprises providing the medical context information (202) to the medical diagnosis tool for performing the medical diagnosis.

13. The method (100) according to any one of the preceding claims, wherein the method further comprises a further training of the language-image model using the medical image (200) and the text query (206).

14. A computer system (402) configured for providing a value (212) of an image quality metric of at least one medical image (200) using a trained language-image model,
wherein the computer system (402) comprises a memory unit (405) storing machine executable instructions and the trained language-image model, wherein execution of the machine-readable instructions causes the computer system (402) to:
- receive (102) the medical image (200) and medical context information (202) associated with the medical image, wherein the medical context information is descriptive of a subject, a medical exam performed on the subject, and/or a medical imaging system (300) which performed the medical exam to acquire the medical image;
- provide (104) the medical image (200) to a source encoder (204) of the language-image model, wherein the source encoder is configured to encode the medical image into an image embedding in an embedding space;
- generate (106) a text query (206) descriptive of the medical image (200) using the medical context information (202) and/or the image quality metric;
- provide (108) the text query (206) to a query encoder (208) of the language-image model, wherein the query encoder is configured to encode the text query into a text embedding in the embedding space;
- determine (110) a similarity (210) between the image embedding and the text embedding in the embedding space;
- determine (112) the value (212) of the image quality metric using the similarity (210); and
- provide (114) the value (212) of the image quality metric.

15. A computer program product comprising machine executable instructions configured for causing a computer system (402) to perform the method (100) of claims 1 through 13.
